Europäisches Patentamt

European Patent Office

Office européen des brevets

(19)

(11) Numéro de publication: **0 364 801**
**A1**

(12) ## DEMANDE DE BREVET EUROPEEN

(21) Numéro de dépôt: 89118233.9

(51) Int. Cl.5: **A61F 9/00**

(22) Date de dépôt: 02.10.89

(30) Priorité: 06.10.88 CH 3745/88
06.10.88 FR 8813397

(43) Date de publication de la demande:
25.04.90 Bulletin 90/17

(84) Etats contractants désignés:
AT BE DE GB IT LU NL SE

(71) Demandeur: LASAG AG
Mittlere Strasse 52
CH-3600 Thun(CH)

(72) Inventeur: Dürr, Ulrich
Ziegeleistrasse 28
CH-3612 Steffisburg(CH)
Inventeur: Taoufik, Nouri
Oberstadelstrasse 14
CH-3653 Oberhofen(CH)
Inventeur: Steinger, Jürg
Hämlismatt
CH-3508 Arnisäge(CH)

(74) Mandataire: de Montmollin, Henri et al
ICB Ingénieurs Conseils en Brevets SA
Passage Max. Meuron 6
CH-2001 Neuchâtel(CH)

(54) Dispositif pour le traitement chirurgical d'un point situé dans un oeil.

(57) Le dispositif comporte un premier générateur (1) destiné à produire un premier faisceau de lumière cohérente (2) dont l'énergie est concentrée dans une succession d'impulsions susceptibles, chacune, de provoquer une microexplosion dans le tissu organique situé au point à traiter, un deuxième générateur (8) destiné à produire un deuxième faisceau de lumière cohérente (9) susceptible de pénétrer dans l'épaisseur de ce tissu organique, un système optique (3,6) destiné à focaliser le premier ou le deuxième faisceau (9) sur le point à traiter, et un commutateur de faisceau (10) susceptible de diriger sélectivement le premier (2) ou le deuxième faisceau (9) vers le système optique (3,6).

Le deuxième faisceau donne aux ophtalmologues la possibilité de réaliser des opérations qui n'étaient pas réalisables avec les dispositifs connus, notamment des opérations nécessitant une pénétration du faisceau de traitement dans l'épaisseur des tissus de l'oeil.

FIG. 1

## DISPOSITIF POUR LE TRAITEMENT CHIRURGICAL D'UN POINT SITUE DANS UN OEIL

Les dispositifs pour le traitement chirurgical d'un point situé dans un oeil qui sont disponibles actuellement comportent généralement deux générateurs produisant, chacun, un faisceau de lumière cohérente.

Le premier de ces générateurs comporte un laser de puissance, par exemple un laser dont le milieu actif est un barreau de grenat d'yttrium et d'aluminium dopé au néodyme (appelé généralement Nd:YAG laser) qui produit un faisceau de lumière cohérente infrarouge ayant une longueur d'onde de 1,06 micromètre. Ce laser est associé à ce que les spécialistes appellent un "Q-switch", c'est-à-dire un commutateur de facteur de qualité.

Ce "Q-switch" est constitué par une cellule électro-optique, par exemple une cellule de Pockels, disposée dans la cavité résonnante du laser et capable de modifier le facteur de qualité Q de cette cavité résonnante (d'où son nom de Q-switch) de manière que toute l'énergie lumineuse produite par le laser soit concentrée dans des impulsions successives très courtes, ayant une durée de quelques nanosecondes.

Lorsqu'un faisceau de lumière cohérente produit par un tel générateur est focalisé sur un point à traiter dans un oeil, chaque impulsion provoque une micro-explosion qui détruit la matière présente en ce point.

Le deuxième générateur des dispositifs connus comporte un laser dans la cavité résonnante duquel les ondes lumineuses peuvent osciller librement, c'est-à-dire ne comportant pas de "Q-switch".

Le faisceau de lumière cohérente produit par ce deuxième générateur est essentiellement destiné à coaguler le sang qui peut s'épancher dans l'oeil. Le milieu actif du laser de ce deuxième générateur est donc choisi de manière que cette lumière cohérente soit bien absorbée par les pigments rouges du sang. Un tel milieu actif est, par exemple, de l'argon, auquel cas la lumière cohérente émise a une longueur d'onde comprise entre 488 et 514 nanomètres, c'est-à-dire une couleur allant du bleu au vert, qui peut être sélectionnée par l'ophtalmologue.

Du fait qu'il est absorbé par le sang, qui est présent dans tous les tissus de l'oeil, le faisceau de lumière cohérente produit par le deuxième générateur des dispositifs connus n'a qu'une profondeur de pénétration très faible dans ces tissus. Il ne peut donc pas être utilisé lorsque l'opération à réaliser nécessite une pénétration du faisceau de lumière cohérente jusqu'à une profondeur relativement grande dans le tissu de l'oeil.

Des recherches en laboratoire et des essais cliniques ont montré que de telles opérations peuvent être avantageusement réalisées à l'aide d'un faisceau de lumière cohérente ayant une longueur d'onde semblable à celle de la lumière émise par les lasers dont le milieu actif est un barreau en grenat d'yttrium et d'aluminium dopé au néodyme, lorsque ce faisceau est continu ou formé d'impulsions relativement longues, c'est-à-dire lorsque le laser produisant ce faisceau n'est pas associé à un "Q-switch".

On peut consulter à ce sujet, par exemple, le livre intitulé "Neodymium : YAG Laser Microsurgery : Fundamental Principles and Clinical Applications", édité pr M. R. M. Klapper aux Editions Little, Brown and Co à Boston (USA), et plus particulièrement les chapitres "Microsurgery with the Neodymium : YAG Laser : An Overview" écrit par MM. F. Fankhauser et P. Rol, et "Neodymium : YAG Laser Capsulotomy" écrit par MM. T. A. Deutsch et M. F. Goldberg.

Pour réaliser toutes les opérations de microchirurgie possibles, un ophtalmologue devrait donc disposer d'une installation comprenant, d'une part, un dispositif connu tel que celui qui a été décrit ci-dessus et permettant de réaliser les opérations classiques, et, d'autre part, une générateur produisant un faisceau continu ou pulsé de lumière cohérente à grande longueur d'onde, avec un système optique adapté à ce générateur, permettant de réaliser les opérations mentionnées, par exemple, dans le livre cité ci-dessus.

Une telle installation serait encombrante et chère.

Un but de la présente invention est de proposer un dispositif permettant à un ophtalmologue de réaliser toutes les opérations de microchirurgie opthalmologique qu'il peut désirer réaliser, ce dispositif étant moins encombrant et meilleur marché que l'installation mentionnée ci-dessus.

Ce but est atteint par le dispositif revendiqué, qui comprend un premier générateur destiné à produire un premier faisceau de lumière cohérente et comportant des moyens pour concentrer l'énergie dudit premier faisceau dans une succession d'impulsions susceptibles, chacune, de provoquer une microexplosion dans la matière située audit point, et un système optique destiné à focaliser ledit premier faisceau sur ledit point, et qui est caractérisé par le fait qu'il comporte un deuxième générateur destiné à produire un deuxième faisceau de lumière cohérente susceptible de pénétrer dans l'épaisseur de ladite matière, et un commutateur de faisceau susceptible de diriger sélectivement ledit premier faisceau et ledit deuxième faisceau dans l'axe dudit système optique.

D'autres caractéristiques et avantages de l'invention ressortiront de la description qui va être faite ci-après à l'aide du dessin annexé dans lequel :

- la figure 1 est une représentation schématique d'une forme d'exécution du dispositif selon l'invention ;

- la figure 2 est une schéma d'un exemple de circuit de commande du dispositif de la figure 1 ;

- la figure 3 illustre une autre forme d'exécution du dispositif selon l'invention ;

- la figure 4 est une représentation schématique d'une autre forme d'exécution du dispositif selon l'invention ; et

- la figure 5 illustre une autre forme d'exécution du dispositif selon l'invention.

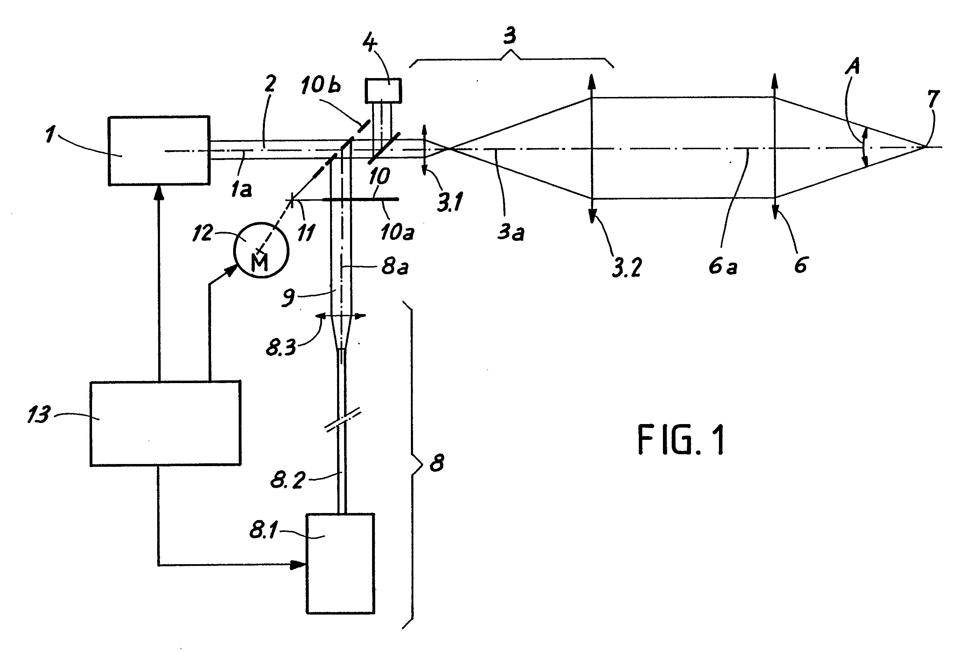

Dans sa forme d'exécution représentée schématiquement et à titre d'exemple non limitatif à la figure 1, le dispositif selon l'invention comporte une premier générateur, désigné par la référence 1.

Ce générateur 1 est destiné à produire un faisceau de lumière cohérente 2 selon un axe d'émission 1a, ce faisceau 2 étant constitué d'impulsions très courtes et très intenses capables de provoquer des microexplosions au point où il est focalisé et de détruire les tissus organiques présents en ce point, ou toute autre matière affectant le bon fonctionnement de l'oeil.

Comme dans les dispositifs connus, ce générateur 1 peut comporter un laser dont le milieu actif est constitué par un barreau en grenat d'yttrium et d'aluminium dopé au néodyme (Nd : YAG laser) associé à un commutateur de facteur de qualité (Q-switch), et il ne sera pas décrit en détail ici.

Le dispositif de la figure 1 comporte également un télescope 3 dont l'axe optique 3a coïncide avec l'axe d'émission 1a du générateur 1, ainsi qu'un système de mesure d'énergie 4, réalisé par exemple au moyen d'une boule de Ulbricht et placé en amont du télescope 3.

De manière bien connue, le télescope 3 est constitué de deux systèmes optiques symbolisés chacun par une simple lentille convergente et désignés par les références 3.1 et 3.2.

Le dispositif de la figure 1 comporte encore un objectif 6, également symbolisé par une simple lentille convergente, dont l'axe optique 6a coïncide avec l'axe optique 3a du télescope et, donc, avec l'axe d'émission 1a du générateur 1.

Le dispositif de la figure 1 comporte en outre des moyens, non représentés, qui permettent de déplacer le boîtier, non représenté, dans lequel sont disposés le générateur 1, le télescope 3 et l'objectif 6, afin de faire coïncider le foyer 7 de l'objectif 6 avec le point à traiter. Le cas échéant des moyens permettant de déplacer l'objectif 6 par rapport au boîtier pourrait également être prévus.

Comme les dispositifs connus mentionnés ci-dessus, le dispositif de la figure 1 comporte un deuxième générateur, désigné par la référence 8. Mais contrairement aux deuxièmes générateurs des dispositifs connus, le générateur 8 est destiné à produire un faisceau de lumière cohérente 9 ayant une longueur d'onde assez élevée pour que sa profondeur de pénétration dans les tissus de l'oeil soit importante.

Par exemple, le générateur 8 peut comporter un laser dont le milieu actif est un barreau de grenat d'yttrium et d'aluminium dopé au néodyme, semblable au laser du générateur 1.

Mais contrairement à ce dernier, le laser du générateur 8 ne comporte pas de commutateur de facteur de qualité (Q-switch), et il est agencé de manière que le faisceau 9 émis selon l'axe d'émission 8a du générateur 8 soit, au choix, continu ou formé d'impulsions ayant une durée de quelques millisecondes.

Du fait de son mode de fonctionnement, le laser du générateur 8, qui est désigné par la référence 8.1 dans la figure 1, dissipe une quantité importante de chaleur lorsqu'il fonctionne.

Ce laser 8.1 ne peut donc pas être monté dans le boîtier, non représenté, qui contient tous les autres éléments du dispositif qui ont été décrits jusqu'ici.

La liaison entre le laser 8.1 et le reste du dispositif est réalisée par une fibre optique 8.2 dont une extrémité est couplée, par des moyens non représentés, au laser 8.1, et dont l'autre extrémité est fixée dans le boîtier mentionné ci-dessus.

Le faisceau de lumière émis par cette autre extrémité de la fibre optique 8.2 est divergent, et le générateur 8 comporte un système optique 8.3 agencé de manière que ce faisceau divergent soit rendu parallèle et forme le faisceau 9.

Dans l'exemple de la figure 1, ce système optique 8.3 est disposé de manière que son axe optique, qui est l'axe d'émission 8a du généteur 8, coupe à angle droit l'axe d'émission 1a du générateur 1.

Le dispositif de la figure 1 comporte encore un commutateur de faisceau symbolisé par un miroir 10 susceptible de prendre deux positions stables 10a et 10b, par exemple en étant entraîné en rotation autour d'un axe 11 par un moteur 12, ou tout autre convertisseur électro-mécanique adéquat.

Lorsqu'il occupe sa position 10a, le miroir 10 est disposé en dehors du trajet du faisceau 2, de sorte que ce dernier, lorsqu'il est émis par le générateur 1, peut atteindre le télescope 3.

Lorsqu'il occupe sa position 10b, le plan du miroir contient le point d'intersection des axes d'émission 1a et 8a, et fait avec chacun de ces derniers un angle de 45° de manière que le faisceau 9, lorsqu'il est émis par le générateur 8, est réfléchi en direction du télescope 3 et que son axe, après cette réflexion, coïncide avec l'axe optique 3a de ce télescope 3.

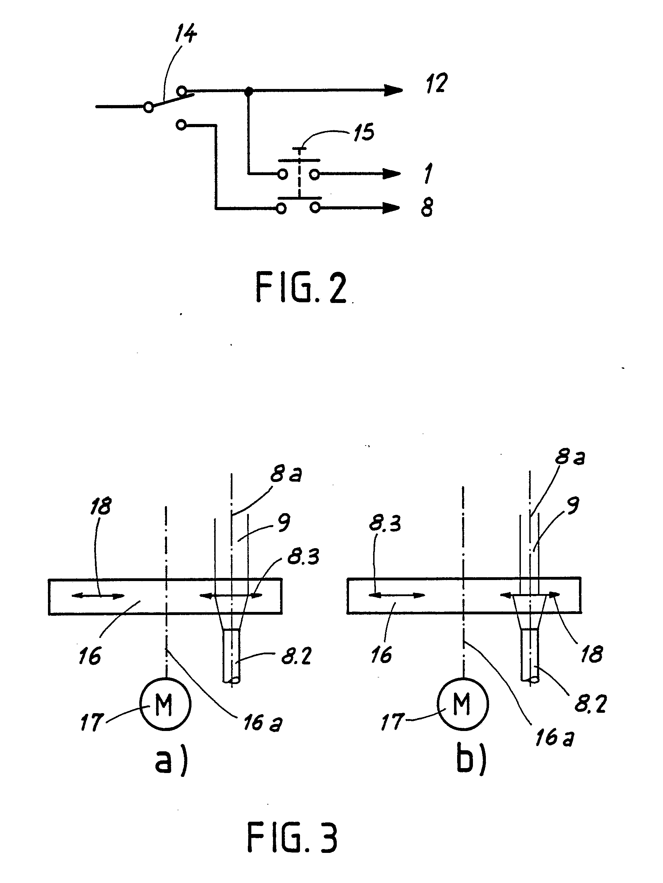

Enfin, le dispositif de la figure 1 comporte un circuit de commande 13 dont un exemple, très simplifié, est représenté à la figure 2.

Ce circuit de commande 13 est relié aux générateurs 1 et 8 ainsi qu'au moteur 12 destiné à déplacer le miroir 10 de l'une à l'autre de ses positions 10a et 10b.

Le circuit de commande 13 comporte un commutateur 14 qui peut être mis manuellement dans l'une ou l'autre de ses deux positions stables et un interrupteur 15 comportant deux contacts qui peuvent être fermés en actionnant, par exemple, un bouton-poussoir, ces deux contacts s'ouvrant à nouveau lorsque ce bouton-poussoir est relaché.

Dans la position où le commutateur 14 est représenté à la figure 2, le moteur 12 d'entraînement du miroir est commandé de manière que ce dernier prenne sa position 10a et, lorsque l'interrupteur 15 est fermé, le générateur 1 est mis en service. Ce générateur 1 produit alors le faisceau 2, qui est élargi dans le télescope 3, puis focalisé par l'objectif 6 sur le point à traiter.

Lorsque le commutateur 14 est mis dans son autre position stable, le moteur 12 d'entraînement du miroir 10 est commandé de manière que ce dernier prenne sa position 10b. Dans ce cas, si l'interrupteur 15 est actionné, c'est le générateur 8 qui est mis en service. Le faisceau 9 qu'il produit est alors réfléchi par le miroir 10 de manière que son axe coïncide avec l'axe optique 3a du télescope 3, élargi par le télescope 3, puis focalisé par l'objectif 6 sur le point à traiter.

En outre, le dispositif de la figure 1 peut comporter des moyens, non représentés, permettant de déplacer le système optique 4 le long de l'axe 3a pour défocaliser le faisceau 2 ou 9 et modifier le diamètre de la zone de l'oeil qu'il atteint, ce qui permet de faire varier la densité de l'énergie lumineuse dans cette zone.

Dans le dispositif de la figure 1, l'angle au sommet du cône formé par le faisceau 2 ou le faisceau 9 lorsqu'il est focalisé par l'objectif 6 est fixe.

De manière bien connue, et pour un télescope 3 et un objectif 6 donnés, cet angle, désigné par A dans la figure 1, ne dépend que du diamètre du faisceau entrant dans le télescope 3.

On admettra que, dans l'exemple de la figure 1, les diamètres des faisceaux 2 et 9 sont égaux et tels que l'angle A vaut 16°, ce qui est une valeur couramment utilisée.

Il est cependant bien connu que, selon le genre d'opération à réaliser et selon l'emplacement du point à traiter dans l'oeil, l'ophtalmologue peut désirer que cet angle ait une valeur plus faible, typiquement 8°, notamment lorsque le faisceau qu'il veut utiliser est le faisceau 9 produit par le générateur 8.

La figure 3 ilustre une forme d'exécution du dispositif selon l'invention dans laquelle le diamètre du faisceau 9 émis par le générateur 8 peut être modifié.

Dans cette forme d'exécution, le système optique 8.3 n'est plus fixe, mais il est disposé sur un support mobile 16 qui peut être entraîné en rotation autour d'un axe 16a par un moteur 17 pour prendre l'une ou l'autre de deux positions angulaires bien définies.

Dans l'une de ces positions, qui est celle qui est représentée à la figure 3a, le système optique 8.3 occupe exactement la même place que celle qu'il a dans le dispositif de la figure 1. Le faisceau 9 a donc le même diamètre que dans ce dernier dispositif, et l'angle A vaut également 16°.

Un deuxième système optique, désigné par 18, est monté sur le support mobile 16 de manière que, lorsque ce dernier occupe sa deuxième position angulaire comme cela est illustré par la figure 3b, ce système optique 18 soit aligné avec la fibre optique 8.2 et reçoive donc le faisceau divergent sortant de cette dernière.

Le système optique 16 est dimensionné de manière que ce faisceau divergent soit rendu parallèle et, en outre, que le diamètre de ce faisceau parallèle, qui constitue dans ce cas, le faisceau 9, ait la valeur nécessaire pour que l'angle A soit de 8°.

Le moteur 15 peut évidement être commandé par l'ophtalmologue, par l'intermédiaire d'un organe de commande adéquat, non représenté, faisant partie du circuit de commande 13.

Les autres éléments de cette forme d'exécution du dispositif selon l'invention sont identiques à ceux qui ont été décrits à l'aide de la figure 1. C'est pourquoi ils n'ont pas été représentés à nouveau dans cette figure 3.

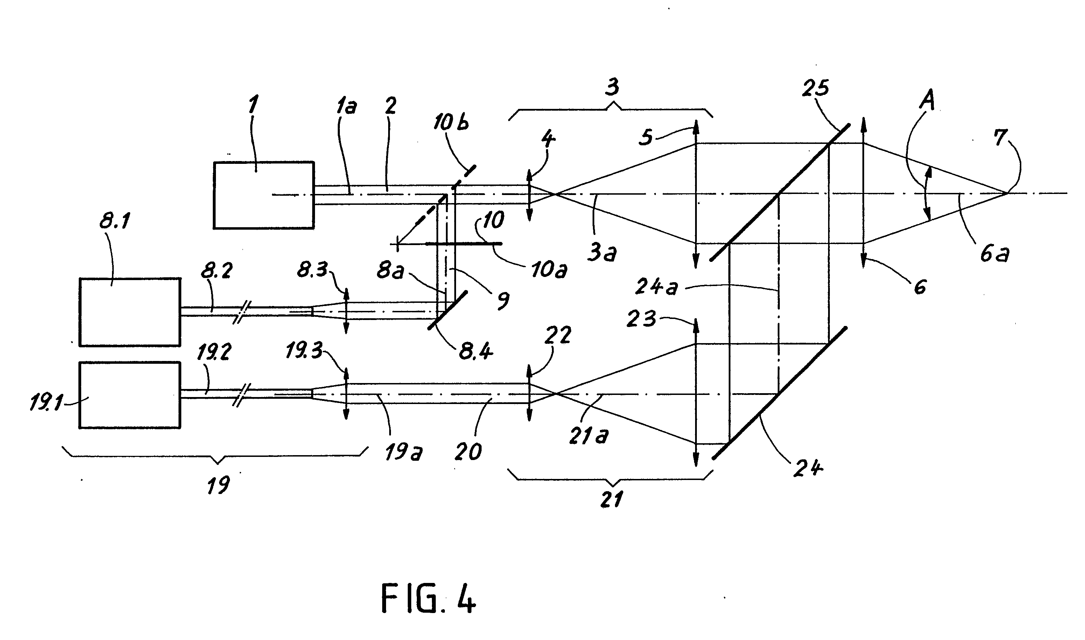

Dans la forme d'exécution représentée à la figure 4, le dispositif selon l'invention comporte les mêmes éléments que le dispositif de la figure 1. Ces éléments sont désignés par les mêmes références que dans cette figure 1, et ils ne seront pas décrits à nouveau ici. Seuls le moteur 12 destiné à déplacer le miroir 10 de l'une à l'autre de ses positions 10a et 10b et le circuit de commande 13 n'ont pas été représentés dans cette figure 4, pour ne pas charger inutilement le dessin.

Outre les éléments mentionnés ci-dessus, le dispositif de la figure 4 comporte un troisième générateur 19 destiné à produire un faisceau de lumière cohérente ayant une longueur d'onde telle qu'elle est bien absorbée par les pigments rouges du sang.

Comme dans les dispositifs connus décrits ci-dessus, ce générateur 19 peut comprendre un laser dont le milieu actif est constitué par de l'argon, la lumière émise par le générateur ayant, dans ce cas, une longueur d'onde comprise entre 488 et 514 nanomètres.

Comme le laser 8.1 du générateur 8, le laser du générateur 19, désigné par 19.1, dissipe une énergie importante lorsqu'il fonctionne et ne peut donc pas être disposé dans le boîtier, non représenté, qui contient les autres éléments du dispositif.

Ce laser 19.1 est donc disposé à l'extérieur de ce boîtier, et il est relié à celui-ci par une fibre optique désignée par 19.2.

Le faisceau divergent de lumière cohérente, qui sort de cette fibre 19.2 est transformé en un faisceau parallèle 20 par un système optique 19.3.

En outre, les éléments du dispositif décrits ci-dessus sont disposés de manière que l'axe optique de système optique 19.3, qui constitue l'axe d'émission 19a du générateur 19, soit parallèle à l'axe d'émission 1a du générateur 1.

Ce faisceau parallèle 20 est dirigé sur un télescope 21 dont l'axe optique 21a est aligné sur l'axe d'émission 19a du générateur 19 et, donc, est parallèle à l'axe optique 3a du télescope 3.

Comme ce dernier, le télescope 21 est formé de deux systèmes optiques désignés par 22 et 23.

Un miroir 24 est disposé sur le chemin optique du faisceau sortant du télescope 21, de manière que son plan fasse un angle de 45° avec l'axe 21a, et que ce faisceau soit réfléchi en direction de l'axe 3a du télescope 3.

L'axe 24a du faisceau réfléchi par ce miroir 24 est donc perpendiculaire à l'axe optique 3a du télescope 3.

Un miroir semi-transparent 25 est disposé entre le télescope 3 et l'objectif 6, de manière que son plan contienne le point d'intersection des axes 3a et 24a, que ce plan fasse un angle de 45° avec ces deux axes, et que le faisceau réfléchi par le miroir 24 soit à nouveau réfléchi en direction de l'objectif 6.

Les faces de ce miroir semi-transparent 25 sont spécialement traitées, de manière bien connue qui ne sera pas décrite ici, pour que le faisceau 2 ou le faisceau 9, après avoir traversé le télescope 3, soit transmis en direction de l'objectif 6 avec le moins de perte possible, et que le faisceau 20, après avoir été réfléchi par le miroir 24, soit à nouveau réfléchi en direction de cet objectif 6 également avec le moins de perte possible.

Dans la forme d'exécution illustrée par la figure 4, la fibre optique 8.2 et le système optique 8.3 sont disposés de manière que l'axe du faisceau sortant de ce système optique 8.3 soit parallèle aux axes d'émission 1a et 19a des générateurs 1 et 19, pour des raisons qui seront données ci-dessous.

Cependant, l'axe d'émission 8a du générateur 8 doit être perpendiculaire à l'axe d'émission 1a du générateur 1 pour que le faisceau 9 puisse être réfléchi en direction du télescope 3 par le miroir 10 lorsque celui-ci occupe sa position 10b. Le générateur 8 comporte donc, dans cette forme d'exécution, un miroir 8.4 dont le plan fait un angle de 45° avec l'axe du système optique 8.3 et qui est disposé de manière que le faisceau sortant de ce système optique forme le faisceau 9 émis par le générateur 8.

On voit que, dans cette forme d'exécution représentée à la figure 4, le dispositif selon l'invention permet à un ophtalmologue de réaliser toutes les opérations qu'il peut souhaiter réaliser dans un oeil, grâce au fait qu'il comporte non seulement les deux générateurs 1 et 19 qui sont semblables aux générateurs que comprennent les dispositifs connus, mais encore le générateur 8 qui, comme dans le dispositif de la figure 1, permet de réaliser des opérations nécessitant une pénétration importante du faisceau de traitement dans le tissu de l'oeil.

Dans l'exemple illustré par la figure 4, les systèmes optiques 8.3 et 19.3 sont conçus de manière que les faisceaux 9 et 20 ont le même diamètre que le faisceau 2, et le télescope 21 a le même grossissement que le télescope 3. Il en résulte que, quel que soit le faisceau utilisé, l'angle au sommet A du cône formé par ce faisceau lorsqu'il est focalisé par l'objectif 6 a toujours la même valeur, par exemple 16°.

Il est évident que, dans une autre forme d'exécution qui n'a pas été représentée, un système semblable à celui qui est représenté à la figure 3 peut être associé avec chacun des générateurs 8 et 19 dans le but de permettre la modification de cet angle A.

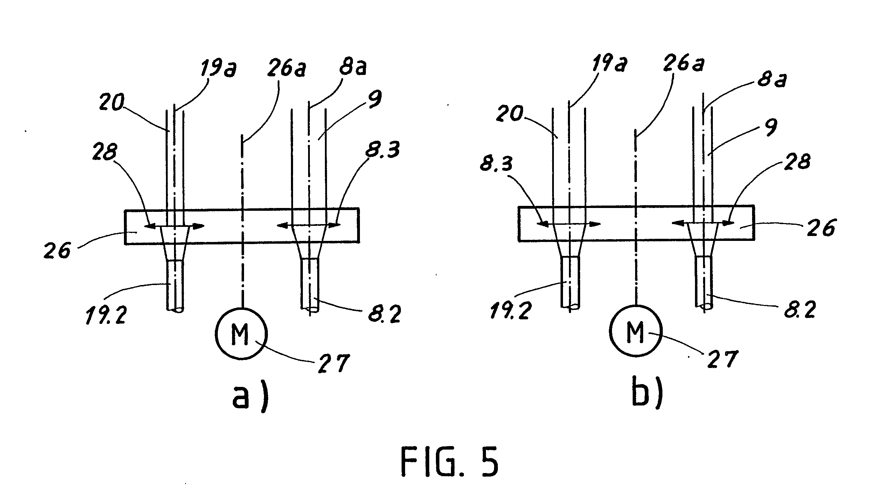

La figure 5 illustre une autre forme d'exécution du dispositif de la figure 4 dans laquelle la modification de cet angle A peut être réalisée de manière plus simple, notamment grâce au fait que la fibre optique 8.2 est disposée comme cela est représenté dans cette figure 4.

Dans cette forme d'exécution, le système optique 8.3 est disposé sur un support mobile semblable au support 16 de la figure 3 et désigné par 26.

Ce support 26 est relié mécaniquement à un moteur 27 qui peut le faire tourner autour d'un axe 26a et prendre l'une ou l'autre de deux positions angulaires déterminées.

Ce moteur 27 peut être commandé par l'ophtalmologue par l'intermédiaire d'un commutateur ou de tout autre moyen adéquat faisant partie d'un circuit de commande du dispositif qui n'a pas été représenté car sa réalisation ne pose aucun problème particulier.

Le support 26 est disposé de manière que, lorsqu'il occupe la position angulaire illustrée par la figure 5a, le système optique 8.3 est situé en regard de la fibre optique 8.2, dans la même position que dans le cas de la figure 4. Le faisceau lumineux divergent sortant de cette fibre 8.2 est donc également transformé en un faisceau parallèle dont le diamètre, qui est également le diamètre du faisceau 9, est le même que dans ce cas de la figure 4, c'est-à-dire un diamètre tel que l'angle A défini ci-dessus vaut 16°.

Un deuxième système optique, désigné par 28, est disposé sur le support 26, de manière que, lorsque ce dernier est dans la position de la figure 5a, il soit situé en regard de la fibre optique 19.2, à l'endroit qu'occupe le système optique 19.3 dans la figure 4.

Le système optique 28 est semblable au système optique 18 de la figure 3. Il transforme donc le faisceau divergent qui sort de la fibre 19.2 en un faisceau parallèle, qui est le faisceau 20 émis par le générateur 19, dont le diamètre est celui qu'il doit avoir pour que l'angle A mentionné ci-dessus ait une valeur de 8°.

Les extrémités des fibres optiques 8.2 et 19.2 et le support 26 sont en outre disposés les uns par rapport aux autres de manière que, lorsque ce support 26 occupe sa deuxième position déterminée, illustrée par la figure 5b, c'est le système optique 8.3 qui est aligné avec la fibre optique 19.2, et le système optique 28 qui est aligné avec la fibre optique 8.2

On voit facilement que, dans ce cas, l'angle A mentionné ci-dessus vaut 8° lorsque le faisceau 9 est émis, et 16° lorsque le faisceau 20 est émis.

Les autres éléments de cette forme d'exécution du dispositif selon l'invention sont identiques à ceux qui ont été décrits dans le cas de la figure 4, et ils n'ont donc pas été représentés à nouveau dans cette figure 5.

On remarquera que, dans le dispositif selon l'invention, la présence d'un commutateur de faisceau symbolisé par le miroir 10 dans les exemples décrits, ainsi que le fait que les générateurs 1 et 8 produisent des faisceaux de lumière cohérente ayant la même longueur d'onde, ou au moins des longueurs d'onde proches l'une de l'autre, permettent de n'utiliser qu'un seul télescope, le télescope 3 dans les exemples décrits, pour élargir les deux faisceaux et les diriger sur l'objectif du dispositif.

Ce télescope 3 peut ainsi être conçu de manière à transmettre ces faisceaux de manière optimale, avec le moins possible de pertes.

En outre, le fait que le dispositif ne comporte qu'un seul télescope pour deux faisceaux différents diminue son prix de revient, malgré l'adjonction de commutateurs.

Comme pour le système optique 4 de la figure 1, il est possible de prévoir des moyens permettant de déplacer les systèmes 4 et 22 du dispositif de la figure 4 le long des axes 3a et 21a respectivement, pour défocaliser le faisceau de traitement.

Comme ces axes 3a et 21a sont parallèles, ces moyens peuvent comprendre un support, non représenté, sur lequel sont disposés les deux systèmes optiques 4 et 22, ce support pouvant être entraîné en translation parallèlement à ces deux axes 3a et 21a.

Il est évident que de nombreuses modifications peuvent être apportées au dispositif selon l'invention, notamment à la disposition relative de ses divers éléments, sans pour autant que le dispositif ainsi modifié sorte du cadre de la présente invention.

On relèvera cependant que, parmi toutes les dispositions possibles, les plus avantageuses sont celles dans lesquelles l'axe d'émission 1a du générateur 1 est aligné sur l'axe optique 3a du télescope, sans élément intermédiaire, et dans lesquelles le commutateur optique symbolisé par le miroir 10 occupe, lorsque le faisceau 2 est émis par le générateur 1, une position telle qu'il est situé entièrement en dehors du trajet optique de ce faisceau 2.

En effet, ces dispositions sont celles où le faisceau 2, qui est celui dans lequel toute l'énergie lumineuse est concentrée dans des impulsions très courtes, rencontre le moins d'éléments optiques et subit donc le moins de pertes.

Comme les dispositifs connus, le dispositif selon l'invention comporte de préférence des moyens permettant à l'ophtalmologue d'éclairer et d'observer le point de l'oeil qu'il doit traiter, ainsi que des moyens permettant à cet ophtalmologue de déterminer avec précision, avant d'enclencher l'émission du faisceau de traitement, le point où ce faisceau sera focalisé, et de vérifier que ce faisceau de traitement ne risque pas de toucher une autre partie de l'oeil.

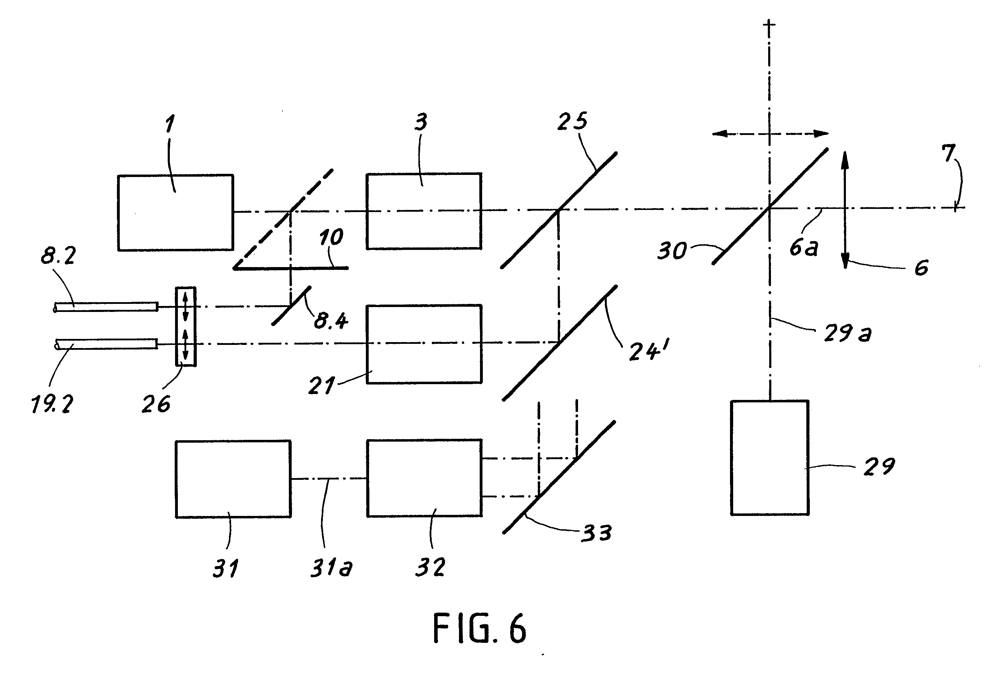

La figure 6 illustre une forme d'exécution du dispositif selon l'invention qui comprend les moyens qui viennent d'être mentionnés, ainsi que pratiquement tous les éléments décrits ci-dessus à l'aide des figures 4 et 5.

Parmi ces éléments, qui portent les mêmes références que dans ces figures 4 et 5 et qui ne seront pas décrits à nouveau ici, seul le miroir 24 a été remplacé par un miroir semi-transparent, désigné par 24', pour une raison qui sera donnée plus loin. En outre, certains éléments, tels les moteurs 12 et 27 ou les lasers 8.1 et 19.1, n'ont pas été représentés dans cette figure 6 pour ne pas la charger inutilement. Pour la même raison, les différents faisceaux des figures 4 et 5, ainsi que les faisceaux qui vont être décrits ci-dessous, sont simplement symbolisés par leurs axes, dessinés en traits mixtes.

Outre les éléments mentionnés ci-dessus, le dispositif de la figure 6 comporte des moyens permettant à l'ophtalmologue d'éclairer et d'observer la zone de l'oeil qu'il doit traiter.

Ces moyens comprennent par exemple une lampe à fente non référencée et un microscope binoculaire 29 qui sont des éléments bien connus et qui ne seront donc pas décrits ici. L'axe optique de ce microscope 29, qui coupe à angle droit l'axe de l'objectif 6, est désigné par 29a.

Un miroir semi-transparent 30 est disposé à l'intersection des axes 6a et 29a, de manière à permettre à l'ophtalmologue d'observer, à travers le microscope, la zone éclairée par la lampe à fente.

Le miroir semi-transparent 30 est bien entendu traité de manière à être aussi transparent que possible pour les faisceaux de traitement 2, 9 et 20, et à être aussi réfléchissant que possible pour la lumière produite par la lampe à fente.

Il faut noter que l'objectif 6 pourrait être disposé de manière que son axe optique coïncide avec l'axe 29a du microscope 29, cette disposition, qui est représentée en traits interrompus à la figure 6, présentant l'avantage que le patient fait face à l'ophtalmologue pendant le traitement.

Dans ce cas, cependant, le miroir 30 doit bien entendu être conçu de manière à être aussi réfléchissant que possible pour les faisceaux de traitement 2, 9 et 20, et à être aussi transparent que possible pour la lumière produite par la lampe à fente.

Le dispositif de la figure 6 comporte en outre des moyens permettant à l'ophtalmologue de déterminer avec précision, avant d'enclencher l'émission du faisceau de traitement 2, 9 ou 20, le point où ce faisceau sera focalisé, et de vérifier si ce faisceau ne risque pas de toucher une autre partie de l'oeil.

De tels moyens, qui sont décrits par exemple dans la demande de brevet EP-A-0 030 210 et ne seront pas décrits en détail ici, comportent un laser de faible puissance, désigné par 31, qui émet un faisceau de lumière visible. Un tel laser peut être un laser dont le milieu actif est un mélange d'hélium et de néon.

Ce faisceau est séparé en deux par un dispositif 32 qui comporte, par exemple, des prismes tournant autour de l'axe d'émission 31a du laser 31, de manière que les faisceaux sortant de ce dispositif 32 soient parallèles et définissent, en tournant autour de leur axe de symétrie, un cylindre ayant le même diamètre que les faisceaux de traitement 2, 9 ou 20.

Un miroir 33 est disposé de manière à réfléchir ces deux faisceaux en direction du miroir 24', qui est un miroir semi-transparent situé à l'emplacement du miroir 24 de la figure 4.

Après avoir traversé ce miroir 24', ces deux faisceaux sont réfléchis par le miroir 25 en direction du miroir 30 et de l'objectif 6.

Ce dernier dévie ces deux faisceaux de manière qu'ils se croisent à son foyer 7.

Comme ces faisceaux tournent autour de leur axe de symétrie, ils définissent, entre l'objectif 6 et le foyer 7, un cône qui est l'enveloppe du faisceau de traitement 2, 9 ou 20.

**Revendications**

1. Dispositif pour le traitement chirurgical d'un point situé dans un oeil, comprenant un premier générateur (1) destiné à produire un premier faisceau de lumière cohérente (2) et comportant des moyens pour concentrer l'énergie dudit premier faisceau (2) dans une succession d'impulsions susceptibles, chacune, de provoquer une microexplosion dans la matière située audit point, et un système optique (3,6) destiné à focaliser ledit premier faisceau (2) sur ledit point, caractérisé par le fait qu'il comporte un deuxième générateur (8) destiné à produire un deuxième faisceau de lumière cohérente (9) susceptible de pénétrer dans l'épaisseur de ladite matière, et un commutateur de faisceau (10) susceptible de diriger sélectivement ledit premier faisceau (2) et ledit deuxième faisceau (10) dans l'axe (3a) dudit système optique (3,6).

2. Dispositif selon la revendication 1, caractérisé par le fait que ledit commutateur de faisceau (10) comporte un élément réfléchissant (10) susceptible de prendre une première position (10a) dans laquelle il est situé entièrement en dehors du chemin optique dudit premier faisceau (2) et une deuxième position (10b) dans laquelle il réfléchit ledit deuxième faisceau (9) en direction dudit système optique (3,6).

3. Dispositif selon la revendication 1, caractérisé par le fait qu'il comporte des moyens (13) pour produire un signal de commande susceptible de prendre un premier ou un deuxième état, que ledit premier générateur (1) répond sélectivement audit premier état du signal de commande pour produire ledit premier faisceau (2), que ledit deuxième générateur (8) répond sélectivement audit deuxième état du signal de commande pour produire ledit deuxième faisceau (9), et que ledit commutateur de faisceau (10) répond sélectivement auxdits premier et deuxième état du signal de commande pour diriger respectivement ledit premier faisceau (2) et ledit deuxième faisceau (9) en direction dudit système optique (3,6).

4. Dispositif selon la revendication 1, caractérisé par le fait qu'il comporte des moyens (16,8.3,18) pour modifier le diamètre dudit deuxième faisceau (9).

5. Dispositif selon la revendication 4, caractérisé par le fait que lesdits moyens (16,8.3,18) pour modifier le diamètre du deuxième faisceau (9) comportent un premier (8.3) et un deuxième élément optique (18) disposés sur un support commun (16) susceptible de prendre l'une ou l'autre de deux positions déterminées dans chacune desquelles un desdits éléments optiques (8.3,18) est disposé sur le trajet dudit deuxième faisceau, ledit premier (8.3) et ledit deuxième élément optique (18) étant conçus de manière à donner respectivement audit deuxième faisceau un premier diamètre et un deuxième diamètre différent dudit premier diamètre.

6. Dispositif selon la revendication 1, caractérisé par le fait qu'il comporte un troisième générateur (19) destiné à produire un troisième faisceau de lumière cohérente (20) ayant une longueur d'onde telle qu'elle est absorbée par les pigments du sang, et des moyens (21,24,25) pour focaliser ledit troisième faisceau (20) sur ledit point.

7. Dispositif selon la revendication 6, caractérisé par le fait que ledit système optique (3,6) comporte un premier télescope (3) et un objectif (6) disposé entre ledit télescope (3) et ledit point à traiter, et que lesdits moyens (21,24,25) pour focaliser ledit troisième faisceau (20) comportent un miroir semi-transparent (25) disposé entre ledit premier télescope (3) et ledit objectif (6) pour diriger ledit troisième faisceau (20) vers ledit objectif (6), et des moyens pour diriger ledit troisième faisceau (20) vers ledit miroir semi-transparent (25), ledit miroir semi-transparent (25) étant conçu de manière à être principalement transparent pour la lumière dudit premier (2) ou dudit deuxième faisceau (9) et à être principalement réfléchissant pour la lumière dudit troisième faisceau (20).

8. Dispositif selon la revendication 6, caractérisé par le fait qu'il comporte des moyens (26,8.3,28) pour modifier le diamètre dudit troisième faisceau (20).

9. Dispositif selon la revendication 8, caractérisé par le fait que lesdits moyens (26,8.3,28) pour modifier le diamètre du troisième faisceau (20) comportent un premier (8.3) et un deuxième élément optique (28) disposés sur un support commun (26) susceptible de prendre l'une ou l'autre de deux positions déterminées dans chacune desquelles un desdits éléments optiques (8.3,28) est disposé sur le trajet dudit troisième faisceau (20), ledit premier (8.3) et ledit deuxième élément optique (28) étant conçus de manière à donner respectivement audit troisième faisceau (20) un premier diamètre et un deuxième diamètre différent dudit premier diamètre.

10. Dispositif selon la revendication 9, caractérisé par le fait que ledit support (26) et lesdits deuxième (8) et troisième (19) générateurs sont disposés de manière que, lorsque ledit support (26) occupe l'une desdites positions déterminées, ledit premier (8.3) et ledit deuxième élément optique (28) sont respectivement disposés sur le trajet dudit deuxième (9) et dudit troisième faisceau (20), et que, lorsque ledit support (26) occupe l'autre desdites positions déterminées, ledit premier (8.3) et ledit deuxième élément optique (28) sont respectivement disposés sur le trajet dudit troisième (20) et dudit deuxième faisceau (9).

FIG. 1

FIG. 2

FIG. 3

FIG. 4

FIG. 5

EP 0 364 801 A1

FIG. 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | WO-A-8 705 794 (ASEREX LTD)<br>* Résumé * | 1-10 | A 61 F 9/00 |
| A | US-A-4 520 816 (SCHACHAR et al.)<br>* Figures 2-3 * | 1-10 | |
| A | US-A-4 373 467 (RICH et al.)<br>* Figures 1,2; colonne 1, lignes 27-33 * | 1 | |

DOMAINES TECHNIQUES
RECHERCHES (Int. Cl.5)

A 61 F
A 61 B

Le présent rapport a été établi pour toutes les revendications

| Lieu de la recherche | Date d'achèvement de la recherche | Examinateur |
|---|---|---|
| LA HAYE | 15-01-1990 | ARGENTINI A. |

EPO FORM 1503 03.82 (P0402)